(19) Europäisches Patentamt
European Patent Office
Office européen des brevets

(11) Veröffentlichungsnummer: **0 294 681 B1**

(12) **EUROPÄISCHE PATENTSCHRIFT**

(45) Veröffentlichungstag der Patentschrift: **02.03.94**

(51) Int. Cl.[5]: **G03C 7/32**, C07D 487/04, G03C 1/10

(21) Anmeldenummer: **88108645.8**

(22) Anmeldetag: **31.05.88**

(54) **Purpurkupplermonomer, polymerer Purpurkuppler und farbfotografisches Aufzeichnungsmaterial,das den polymeren Purpurkuppler enthält.**

(30) Priorität: **11.06.87 DE 3719401**
**08.07.87 DE 3722497**

(43) Veröffentlichungstag der Anmeldung:
**14.12.88 Patentblatt 88/50**

(45) Bekanntmachung des Hinweises auf die Patenterteilung:
**02.03.94 Patentblatt 94/09**

(84) Benannte Vertragsstaaten:
**BE DE FR GB**

(56) Entgegenhaltungen:
**EP-A- 0 219 033**
**DE-A- 3 633 364**
**JP-A-62 019 849**
**US-A- 4 576 910**
**US-A- 4 755 455**

**RESEARCH DISCLOSURE no. 257, September 1985, S. 481-499, Emsworth, Hampshire, GB; disclosure no. 25758 "Color photographic light-sensitive material"**

**PATENT ABSTRACTS OF JAPAN Band 10, Nr. 223 (P-483)(2279), 5. August 1986; & JP-A-6159336**

(73) Patentinhaber: **Agfa-Gevaert AG**
**Kaiser-Wilhelm-Allee**
**D-51373 Leverkusen(DE)**

(72) Erfinder: **Helling, Günter, Dr.**
**In der Hildscheid 16**
**D-5068 Odenthal(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 294 681 B1

## Beschreibung

Die Erfindung betrifft ein neues Purpurkupplermonomer, einen daraus erhaltenen polymeren Purpurkuppler und ein farbfotografisches Aufzeichnungsmaterial, das diesen polymeren Purpurkuppler enthält. Der polymere Purpurkuppler ist durch Polymerisation ethylenisch ungesättigter Monomerer erhalten worden und weist Pyrazoloazolgruppen und Säuregruppen auf.

Es ist bekannt, farbige fotografische Bilder durch chromogene Entwicklung herzustellen, d. h. dadurch, daß man bildmäßig belichtete Silberhalogenidemulsionsschichten in Gegenwart geeigneter Farbkuppler mittels geeigneter farbbildender Entwicklersubstanzen -sogenannter Farbentwickler -entwickelt, wobei das in Übereinstimmung mit dem Silberbild entstehende Oxidationsprodukt der Entwicklersubstanzen mit dem Farbkuppler unter Bildung eines Farbstoffbildes reagiert. Als Farbentwickler werden gewöhnlich aromatische, primäre Aminogruppen enthaltende Verbindungen, insbesondere solche vom p-Phenylendiamintyp, verwendet.

Es ist bekannt, die Farbkuppler in Form von Polymerisatdispersionen zu verwenden, in denen der funktionelle Rest eines Farbkupplers ein- oder mehrfach mit einem Polymergerüst verknüpft und auf diese Weise diffusionsfest gemacht ist. Ein solches Polymer weist somit wiederkehrende Struktureinheiten mit dem Farbkuppler auf.

Farbkuppler, die in Form solcher Polymerisatdispersionen in die Schichten von fotografischen Aufzeichnungsmaterialien eingearbeitet sind, sind im allgemeinen ausreichend diffusionsfest und beinträchtigen die mechanischen Eigenschaften der Schichten auch bei geringen Bindemittelgehalten nur wenig. Es ist besonders wichtig, daß sie bei der Lagerung nicht auskristallisieren, eine gute Stabilität gegen Licht, Wärme und Feuchtigkeit aufweisen und daß auch die daraus erzeugten Farbstoffe stabil sind, die erwünschten spektralen Eigenschaften aufweisen und bei der Entwicklung diffusionsfest und als möglichst feines Korn abgeschieden werden. Farbkuppler, die in Form solcher Polymerisatdispersionen mit einem Molekulargewicht größer als 5000 in fotografische Aufzeichnungsmaterialien eingearbeitet sind, weisen allgemein gute Kolloidstabilität auf und erfüllen einige der genannten an sie gerichteten Forderungen recht gut. Solche hochmolekularen Farbkuppler sind beispielsweise beschrieben in
DE-C-1 297 417, DE-A-24 07 569, DE-A-31 48 125, DE-A-32 17 200, DE-A-33 20 079, DE-A-33 24 932, DE-A-33 31 743, DE-A-33 40 376, DE-A-34 61 455, EP-A-27 284, US-A-4 080 211.

Die hochmolekularen Farbkuppler werden in der Regel durch Polymerisation von ethylenisch ungesättigten sogenannten monomeren Farbkupplern hergestellt.

Die bekannten polymeren Purpurkuppler weisen aber folgende Nachteile auf:
Die aus polymeren Pyrazolon-Kupplern erhaltenen Purpurfarbstoffe weisen eine nicht ideale Absorption auf: Besonders störend ist die Gelbnebendichte, die eine zusätzliche Verwendung von Maskenkupplern erforderlich macht.

Polymere Pyrazoloazol-Kuppler wie z. B. die Verbindungen M-23 bis M-27 aus DE-A-35 16 996 oder Verbindung XV aus EP-A-0 133 262 wie auch die Verbindungen aus US-A-4 576 910 liefern eine völlig unzureichende Farbausbeute und sind aus diesem Grund nicht brauchbar. In der japanischen Patentanmeldung 62-19849 ist als Verbindung M-52 ein Pyrazoloazol-Kuppler beschrieben, der in der Kupplungsstelle eine p-Carboxyphenoxy-Gruppe enthält.

Der vorliegenden Erfindung liegt die Aufgabe zugrunde, für farbfotografische Aufzeichnungsmaterialien verbesserte polymere Purpurkuppler zur Verfügung zu stellen.

Gegenstand der Erfindung sind Purpurkuppler mit mindestens einer kupplungsfähigen Struktureinheit der Formel I

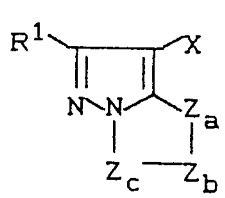

I

worin bedeuten

| | |
|---|---|
| $R^1$ | H, Alkyl, Aralkyl oder Aryl; |
| X | H oder eine durch Kupplung freisetzbare Gruppe; |
| $Z_a$, $Z_b$, $Z_c$ | eine gegebenenfalls substituierte Methingruppe, =N- oder -NH-, wobei entweder die Bindung $Z_a$ - $Z_b$ oder die Bindung $Z_b$ - $Z_c$ eine |

Doppelbindung und die jeweils andere Bindung eine Einfachbindung ist;
und worin mindestens $R^1$ oder X oder ein Substituent an einer durch $Z_a$, $Z_b$ oder $Z_c$ dargestellten Methingruppe eine ethylenisch ungesättigte polymerisierbare Gruppe in gegebenenfalls polymerisierter Form enthält, die direkt oder über ein Bindeglied der Formel

$$-(L^0)_k-(L^1)_l-(L^2)_m-(L^3)_n-(L^4)_o-(L^5)_p-(L^6)_q-(L^7)_r-$$

an einen der beiden heterocyclischen Ringe gebunden ist, worin $L^0$ den dem heterocyclischen Ring und $L^7$ den der ethylenisch ungesättigten Gruppe zunächst gelegenen Teil des Bindegliedes bezeichnen und worin bedeuten (gleich oder verschieden):

| | |
|---|---|
| $L^0$, $L^2$, $L^4$, $L^6$ | -O-, -NR-, -NRCO-, -CONR-, $-NRSO_2-$, $-SO_2NR-$, -COO-, -O-CO-, -NR-CO-NR-, -O-CO-NR-, -NH-COO- (mit R = H oder Alkyl, das gegebenenfalls mit Carboxyl substituiert sein kann.); |
| $L^1$, $L^3$, $L^5$, $L^7$ | Alkylen, Aralkylen, Arylen, gegebenenfalls mit Carboxy substituiert; |
| k,l,m,n,o,p,q,r | jeweils 0 oder 1, wobei gilt, daß |

$$l - m + n - o + p - q = 0,$$

dadurch gekennzeichnet, daß mindestens $R^1$ oder X oder ein Substituent an einer durch $Z_a$, $Z_b$ oder $Z_c$ dargestellten Methingruppe eine Carboxylgruppe enthält, mit der Maßgabe, daß wenn die ethylenisch ungesättigte polymerisierbare Gruppe in $R^1$ oder in einem Substituenten an einer durch $Z_a$, $Z_b$ oder $Z_c$ dargestellten Methingruppe enthalten ist, mindenstens eins von $L^0$, $L^2$, $L^4$ und $L^6$ ein N-Atom mit einer daran gebundenen Carboxyalkylgruppe enthält oder ein C-Atom der ethylenisch ungesättigten polymerisierbaren Gruppe mit einer Carboxylgruppe oder einer Carboxyalkylgruppe substituiert ist.

Bei den Purpurkupplern der Formel I kann es sich gleichermaßen um niedermolekulare polymerisationsfähige Kupplermonomere (nachfolgend als kupplerhaltiges Monomer K bezeichnet) handeln wie auch um polymere Farbkuppler, die durch Polymerisation der Monomere erhalten worden sind (nachfolgend als Polymerkuppler P bezeichnet). Im ersteren Fall ist die erwähnte ethylenisch ungesättigte polymerisierbare Gruppe als solche noch vorhanden; im zweiten Fall ist sie durch Polymerisation in eine Polymerkette einbezogen, wobei das entstandene Polymer die kupplungsfähigen Pyrazoloazolgruppen als Seitengruppen trägt. In jedem Fall ist pro kupplungsfähiger Gruppe mindestens eine Carboxylgruppe vorhanden.

Das kupplerhaltige Monomer K entspricht der allgemeinen Formel I

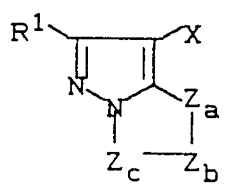

I

worin bedeuten

| | |
|---|---|
| $R^1$ | H, Alkyl, Aralkyl oder Aryl; |
| X | H oder eine durch Kupplung freisetzbare Gruppe; |
| $Z_a$, $Z_b$, $Z_c$ | eine gegebenenfalls substituierte Methingruppe, =N- oder -NH-, wobei entweder die Bindung $Z_a$ - $Z_b$ oder die Bindung $Z_b$ - $Z_c$ eine Doppelbindung und die jeweils andere Bindung eine Einfachbindung ist; |

und worin mindestens $R^1$ oder X oder ein Substituent an einer durch $Z_a$, $Z_b$ oder $Z_c$ dargestellten Methingruppe eine ethylenisch ungesättigte polymerisierbare Gruppe der Formel

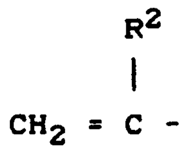

oder

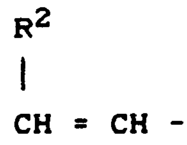

enthält, worin

$R^2$ H, Halogen, insbesondere Chlor, -COOH oder Alkyl, vorzugsweise mit 1 bis 4 C-Atomen und gegebenenfalls mit -COOH substituiert, bedeuten;

wobei weiterhin mindestens $R^1$ oder X oder ein Substituent an einer durch $Z_a$, $Z_b$ oder $Z_c$ dargestellten Methingruppe eine Carboxylgruppe enthält. Vorzugsweise befindet sich die Carboxylgruppe an dem gleichen Substituenten, der auch die polymerisierbare Gruppe enthält.

Eine durch $R^1$ dargestellte Alkyl-, Aralkyl- oder Arylgruppe kann substituiert sein, z. B. mit Halogen, Alkoxy, Aroxy, Acylamino sowie (z. B. im Falle von Aryl) mit Alkyl, und kann desweiteren, gegebenenfalls über derartige Substituenten, die bereits erwähnte ethylenisch ungesättigte polymerisierbare Gruppe sowie gegebenenfalls auch eine Carboxylgruppe enthalten.

Eine durch $Z_a$, $Z_b$ oder $Z_c$ dargestellte substituierte Methingruppe entspricht der Formel

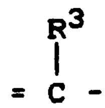

worin $R^3$ für Alkyl mit bis zu 18 C-Atomen, Aryl, Aralkyl, eine heterocyclische aromatische Gruppe, Alkoxy, Amino, einen Carbonamidorest oder -OH steht, wobei insbesondere die genannte Alkyl-, Aralkyl-oder Arylgruppen weiter substituiert sein können und gegebenenfalls über derartige Substituenten die bereits erwähnte ethylenisch ungesättigte polymerisierbare Gruppe und/oder die Carboxylgruppe enthalten können.

Bei der durch X dargestellten freisetzbaren Gruppe handelt es sich beispielsweise um ein Halogenatom z. B. Cl oder um eine organische Gruppe, die in der Regel über ein Sauerstoff-, Schwefel- oder Stickstoffatom an die Kupplungsstelle des Kupplermoleküls angeknüpft ist. Falls es sich bei der abspaltbaren Gruppe um eine cyclische Gruppe handelt, kann die Anknüpfung an die Kupplungsstelle des Kupplermoleküls entweder direkt über ein Atom, das Bestandteil eines Ringes ist, z. B. ein Stickstoffatom, oder indirekt über ein zwischengeschaltetes Bindeglied erfolgt sein. Derartige abspaltbare Gruppen sind in großer Zahl bekannt, z. B. als Fluchtgruppen von 2-Äquivalentpurpurkupplern.

Beispiele von über Sauerstoff angeknüpften abspaltbaren Gruppen entsprechen der Formel

$- O - R^4$,

worin $R^4$ für einen acyclischen oder cyclischen organischen Rest steht, z.B. für Alkyl, Aryl, eine heterocyclische Gruppe oder Acyl, das sich beispielsweise ableitet von einer organischen Carbon- oder Sulfonsäure. Bei besonders bevorzugten abspaltbaren Gruppen dieser Art bedeutet $R^4$ eine gegebenenfalls substituierte Phenylgruppe.

Beispiele von über Stickstoff angeknüpften abspaltbaren Gruppen sind in den folgenden deutschen Offenlegungsschriften (DE-A-) beschrieben:
25 36 191, 27 03 589, 28 13 522, 33 39 201.

Hierbei handelt es sich vielfach um 5-gliedrige heterocyclische Ringe, die über ein Ringstickstoffatom mit der Kupplungsstelle des Purpurkupplers verbunden sind. Die heterocyclischen Ringe enthalten vielfach benachbart zu dem die Bindung an das Kupplermolekül vermittelnden Stickstoffatom aktivierende Gruppen, z. B. Carbonyl- oder Sulfonylgruppen oder Doppelbindungen.

Wenn die abspaltbare Gruppe über ein Schwefelatom an die Kupplungsstelle des Kupplers gebunden ist, kann es sich bei ihr um den Rest einer diffusionsfähigen Mercaptoverbindung handeln, die die Entwicklung von Silberhalogenid zu inhibieren vermag. Derartige Inhibitorreste sind vielfach als an die Kupplungsstelle von Kupplern, auch Purpurkupplern gebundene abspaltbare Gruppe beschrieben worden, z. B. in US-A-3 227 554.

Auch die durch X dargestellte freisetzbare Gruppe kann die erwähnte ethylenisch ungesättigte polymerisierbare Gruppe enthalten, wie beispielsweise beschrieben in Research Disclosure 25724 (September 1985). Ebenso kann die durch X dargestellte freisetzbare Gruppe auch eine Carboxylgruppe enthalten.

Beispiele für kupplerhaltige Monomere K entsprechen den folgenden Formeln II bis VI:

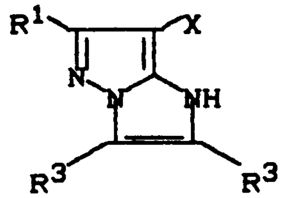

II

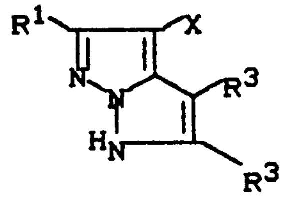

III

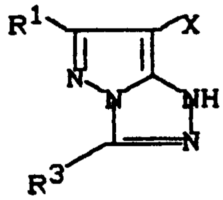

IV

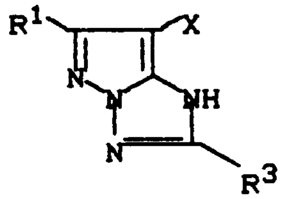

V

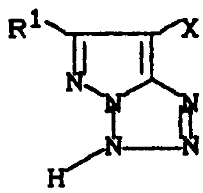

VI

worin X, $R^1$ und $R^3$ die bereits angegebene Bedeutung haben, wobei jedoch zwei in der gleichen Formel (II, III) enthaltene Reste $R^3$ nicht notwendigerweise identisch sind und wobei in jeder der Formeln II bis VI mindestens einer der Reste $R^1$, $R^3$ und X eine ethylenisch ungesättigte polymerisierbare Gruppe und mindestens einer der Reste $R^1$, $R^3$ und X eine Carboxylgruppe enthält. Monomere der Formel IV sind bevorzugt.

Eine durch $L^1$, $L^3$, $L^5$, $L^7$ dargestellte Alkylengruppe kann geradkettig oder verzweigt sein und bis zu 20 C-Atomen aufweisen und gegebenenfalls mit Carboxyl substituiert sein.

Eine durch $L^1$, $L^3$, $L^5$ dargestellte Aralkylengruppe ist beispielsweise eine der folgenden Gruppen;

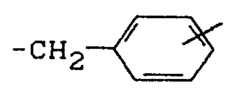 , 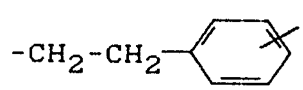 , 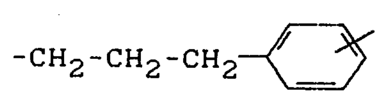

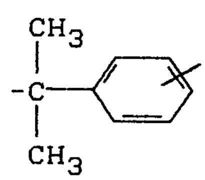 , 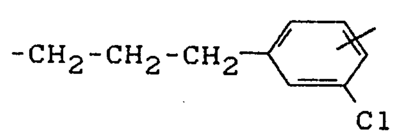 , 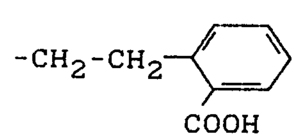

Eine durch $L^1$, $L^3$, $L^5$, $L^7$ dargestellte Arylengruppe ist vorzugsweise eine Phenylengruppe, die substituiert sein kann, z.B. mit Alkyl, Alkoxy, Halogen, Acylamino, Carboxyl oder einem Substituenten, der eine Carboxylgruppe enthält.

Beispiele für geeignete kupplerhaltige Monomere K sind im folgenden angegeben.

K-1

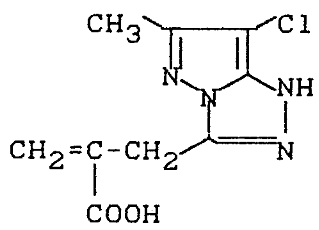

K-2

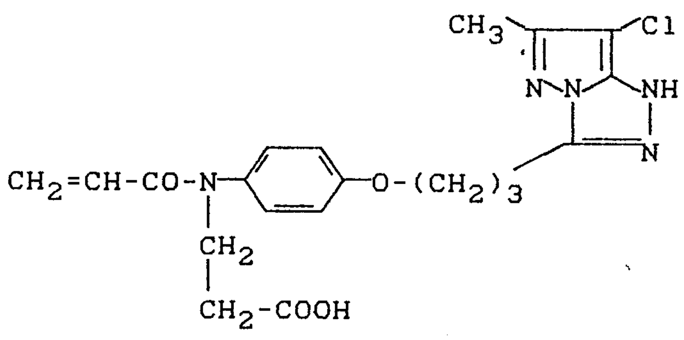

K-3

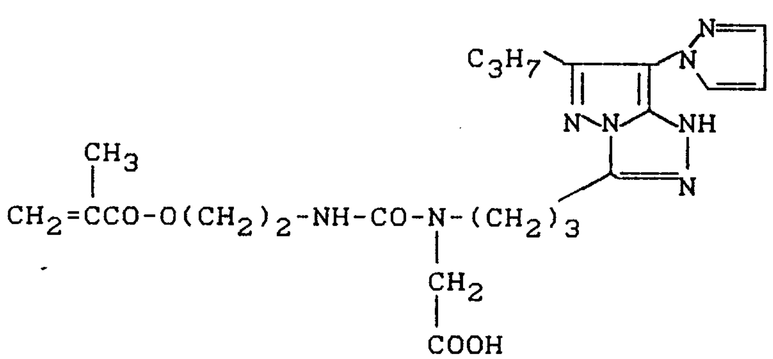

K-5

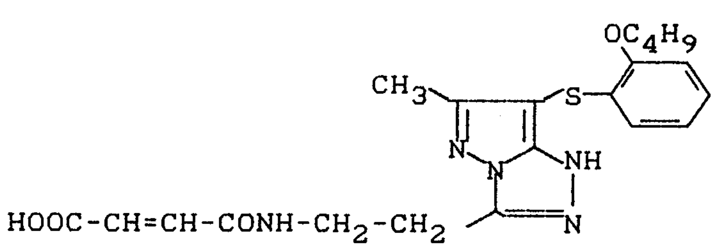

K-6

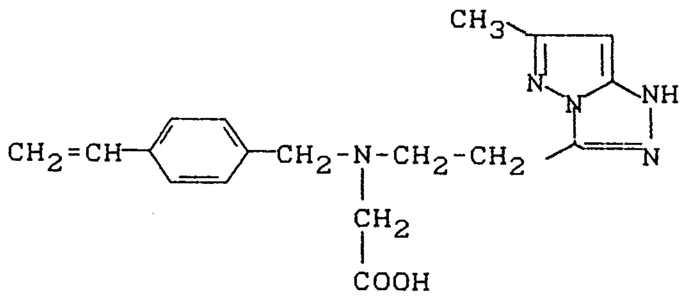

K-7

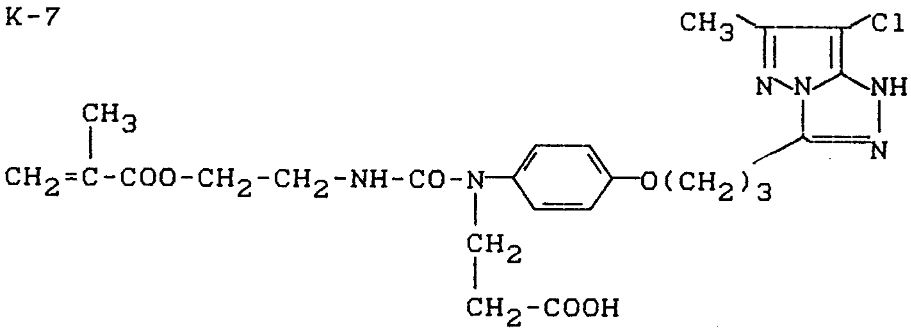

K-10

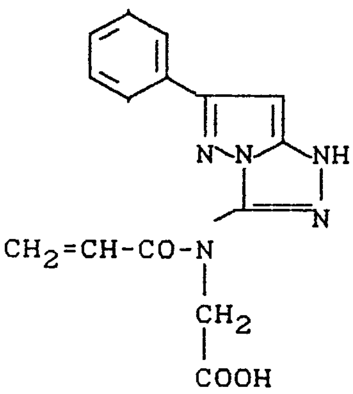

K-11

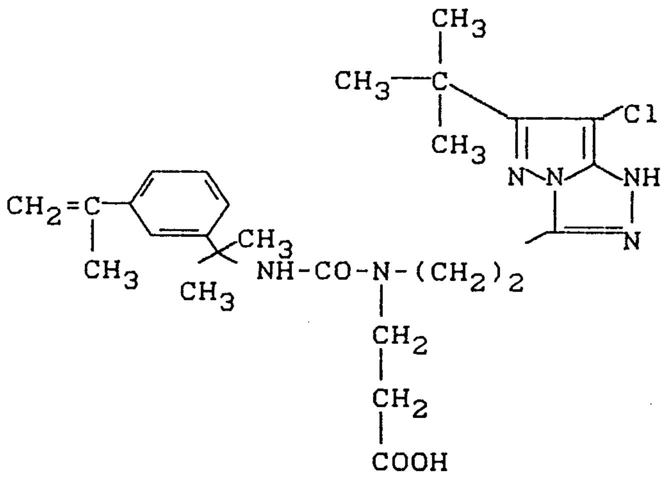

Die erfindungsgemäßen polymeren Farbkuppler werden durch Polymerisation aus den kupplerhaltigen Monomeren K erhalten, wobei weitere polymerisierbare Comonomere anwesend sein können. Sie enthalten mindestens 20 Gew-% an polymerisierten Einheiten der Formel I, d.h. der Anteil des kupplerhaltigen Monomers K in solchen polymeren Farbkupplern beträgt im allgemeinen 20 - 100 Gew.-% vorzugsweise 30 - 70 Gew.-%.

Die erfindungsgemäßen polymeren Kuppler können demnach neben den wiederkehrenden Einheiten aus kupplerhaltigem Monomer K zusätzlich wiederkehrende Einheiten mindestens eines weiteren copolymerisierten Monomers C enthalten. Beispiele für derartige Monomere C umfassen Ester und Amide von Acrylsäure und deren Derivaten, z.B. von Acrylsäure, α-Chloracrylsäure, Methacrylsäure (beispielsweise Acrylamid, Methylmethacrylat, Ethylacrylat, Ethylmethacrylat, n-Propylacrylat, n-Butylacrylat, 2-Ethylhexylacrylat, n-Hexylacrylat, Octylmethacrylat, Laurylmethacrylat und Methylenbisacrylamid), Vinylester (beispielsweise Vinylacetat, Vinylpropionat und Vinyllaurat), Acrylnitril, Methacrylnitril, aromatische Vinylverbindungen (beispielsweise Styrol, Vinyltoluol, Divinylbenzol, Vinylacetophenon, Styrolsulfonsäure), Itaconsäure, Zitraconsäure, Crotonsäure, Methacrylsäure, Vinylidenchlorid, Vinylalkylether (beispielsweise Vinylethylether), Ester von Maleinsäure, N-Vinyl-2-pyrrolidon, N-Vinyl-, 2-Vinyl-und 4-Vinylpyridin. Der Anteil des Monomers C im polymeren Farbkuppler kann 0 - 80 Gew.-%, vorzugsweise 30 -70 Gew.% betragen.

Es ist besonders bevorzugt, als Monomer C einen Ester der Acrylsäure oder Methacrylsäure und/oder eine aromatische Vinylverbindung zu verwenden. Zwei oder mehrere der vorstehend beschriebenen Monomere C können gegebenenfalls gleichzeitig verwendet werden. Beispielsweise ist es möglich, neben dem Monomer K eine Kombination von n-Butylacrylat und Divinylbenzol, Styrol und Methylmethacrylat, Methylacrylat und Butylmethacrylat zu verwenden. Das ethylenisch ungesättigte Monomer C kann ausgewählt werden derart, daß es sich günstig auf die physikalischen Eigenschaften und/oder chemischen Eigenschaften des herzustellenden Copolymers, beispielsweise die Löslichkeit, Verträglichkeit mit einem Bindemittel wie Gelatine oder anderen fotografischen Zusätzen wie farbgebenden Verbindungen, Ultraviolettstrahlen absorbierenden Mitteln, Antioxidantien und dergleichen, sowie auf die Flexibilität der Schichtzusammensetzungen bzw. der farbfotografischen Aufzeich nungsmaterialien auswirkt. Obwohl die Monomere C im allgemeinen keine Gruppen enthalten, die eine fotografische Wirksamkeit entfalten, ist es möglich als Monomer C auch solche Verbindungen zu verwenden, die außer der ethylenisch ungesättigten polymerisierbaren Gruppe eine fotografisch wirksame Gruppe, z. B. eine kupplungsfähige Gruppe, enthalten, die jedoch von der in Formel I dargestellten Kupplergruppe verschieden ist. Solche kupplungsfähigen Gruppen können beispielsweise Weißkuppler-oder Maskenkupplereigenschaften haben oder bei Kupplung einen fotografisch wirksamen Rest, z. B. einen Inhibitor oder Entwicklungsbeschleuniger freisetzen.

Die Molekulargewichte der erfindungsgemäßen Polymere sind vorzugsweise größer als 5000, insbesondere größer als 20.000. Die obere Grenze ist unkritisch und kann, insbesondere wenn als zusätzliches Monomer C bi- oder polyfunktionelle Monomere verwendet werden, Werte von über 10 Millionen erreichen.

Die erfindungsgemäßen polymeren Purpurkuppler werden den farbfotografischen Aufzeichnungsmaterialien in der Regel in Form von Polymerisatdispersionen, z. B. als Polymerlatex zugesetzt.

Die Polymerisation des erfindungsgemäßen Monomers K oder des Monomergemisches (Monomer K und Monomer C) kann nach einem der üblichen Polymerisationsverfahren durchgeführt werden, z. B. durch

Emulsionspolymerisation oder durch Polymerisation in einem organischen Lösungsmittel.

Die Polymerisation der ethylenisch ungesättigten Monomeren wird im allgemeinen initiiert durch freie Radikale, die gebildet werden durch thermische Zersetzung eines chemischen Initiators, durch Einwirkung eines Reduktionsmittels auf eine oxidierende Verbindung (Redox-Initiator) oder durch physikalische Einwirkung, wie Bestrahlung mit Ultraviolettstrahlen oder anderen hochenergetischen Strahlungen, hohen Frequenzen, usw.

Beispiele für chemische Initiatoren umfassen ein Persulfat (beispielsweise Ammoniumpersulfat oder Kaliumpersulfat usw.), ein Peroxid (beispielsweise Wasserstoffperoxid, Benzoylperoxid oder tert. Butylperoctoat), eine Azonitrilverbindung (beispielsweise 4,4′-Azo-bis-4-cyanovaleriansäure oder Azobisisobutyronitril) und einen Azoester (beispielsweise 2,2′-Azobis-(methylisobutyrat)).

Beispiele für konventionelle Redox-Initiatoren umfassen Wasserstoffperoxid-Eisen-(II)-salz, Kaliumpersulfat, Natriummetabisulfit und Cer-IV-salz-Alkohol.

Beispiele für die Initiatoren und deren Funktionen werden beschrieben von F.A. Bovey, in Emulsion Polymerisation, Interscience Publishers Inc., New York, 1955, Seiten 59 bis 93.

Als Emulgator, der bei der Emulsionspolymerisation verwendet werden kann, wird eine Verbindung mit oberflächenaktiver Wirkung verwendet. Bevorzugte Beispiele dafür umfassen Seifen, Sulfonate, Sulfate, kationische Verbindungen, amphotere Verbindungen und hochmolekulare Schutzkolloide. Spezielle Beispiele für die Emulgatoren und deren Funktionen werden beschrieben in Belgische Chemische Industrie, Bd. 28, Seiten 16 bis 20, 1963.

Ein bei der Herstellung des Polymers oder beim Dispergieren des Polymers in einer wäßrigen Gelatinelösung gegebenenfalls verwendetes organisches Lösungsmittel kann vor dem Vergießen aus der Gießlösung entfernt werden.

Als Lösungsmittel kommen beispielsweise solche in Frage, die ein gewisses Ausmaß an Wasserlöslichkeit aufweisen, so daß sie leicht durch Waschen mit Wasser in einem Gelatinenudelzustand entfernt werden können, und solche, die durch Sprühtrocknen, Vakuum- oder Dampfspülen entfernt werden können.

Beispiele solcher Lösungsmittel umfassen Ester (beispielsweise Ethylacetat), Ether, Ketone, halogenierte Kohlenwasserstoffe (beispielsweise Methylenchlorid, Trichlorethylen), Alkohole (beispielsweise Methanol, Ethanol, Butanol) und Kombinationen davon.

Um die Dispersionstabilität zu verbessern und die Flexibilität der aufgeschichteten Emulsion zu verbesseren, kann eine geringe Menge (vorzugsweise nicht mehr als 50 Gew.-% bezogen auf den erfindungsgemäßen polymeren Kuppler) eines permanenten Lösungsmittels, insbesondere eines mit Wasser nicht mischbaren hochsiedenden organischen Lösungsmittels, beispielsweise Dibutylphthalat und/oder Trikresylphosphat, zugesetzt werden. Die Konzentration des permanenten Lösungsmittels soll ausreichend sein, um das Polymer zu plastifizieren, während es in einem Zustand eines festen Teilchens gehalten wird. Andererseits soll die Konzentration des permanenten Lösungsmittels im Interesse einer geringen Schichtdicke und Schichtbelastung so gering wie möglich sein.

Nachstehend werden einige repräsentative Synthesebeispiele für die erfindungsgemäßen Purpurkuppler beschrieben.

Monomer K-2

23 g 3-[3-(4-aminophenoxy)propyl]-6-methyl-7-chlor-1H-pyrazolo[5,1-c]-1,2,4-triazol und 10,8 g Acrylsäure werden in 85 °C warmen Dimethylacetamid gelöst und 1 h bei dieser Temperatur gerührt. Man gibt das Reaktionsgemisch anschließend auf 300 g Eis/Wasser Gemisch, saugt den angefallenen Niederschlag ab und kristallisiert aus Essigester um. Man erhält in 61 %iger Ausbeute 3-[3-(4-carboxyethylaminophenoxy)-propyl]-6-methyl-7-chlor-1H-pyrazolo [5,1-c]-1,2,4-triazol mit einem Schmelzpunkt von 151-153 °C.

23 g diesen Produktes und 4,0 g N,N-Dimethylanilin werden in 100 ml Dimethylacetamid gelöst und auf -12 °C abgekühlt. Dazu tropft man eine Lösung aus 3,2 g Acrylsäurechlorid gelöst in 10 ml Dimethylacetamid zu und rührt 30 min bei 10 °C. Das Reaktionsgemisch wird in 700 g einer Eis/Wasser-Mischung gegeben mit 20 ml einer konzentrierten Salzsäure versetzt und der entstandene Niederschlag abgesaugt. Das erhaltene Rohprodukt wird aus Essigester umkristallisiert. Man erhält in 60 %iger Ausbeute das Monomer K-2 mit einem Schmelzpunkt von 175 °C.

Monomer K-7

13 g 3-[-3-(4-carboxyethylaminophenoxy)-propyl]-6-methyl-7-chlor-1H-pyrazolo[5,1-c]-1,2,4-triazol (siehe Herstellung von Monomer K-2) wurden in 50 ml wasserfreiem Pyridin gelöst und mit 0,16 g Di-tert.-

butylphenol versetzt. Anschließend werden bei 25 °C 5,3 g Isocyanatoethylmethacrylat zugetropft. Nach 30 min Nachrühren wird das Produkt durch Einrühren in Ligroin gefällt, abgesaugt, mit Petrolether nachgewaschen und in Methanol gelöst.

Polymerkuppler P-1

Copolymer aus Monomer K-2 und Ethylacrylat

Unter Stickstoff wurde eine Lösung aus 2,1 g Oleylmethyltaurid in 409 g Wasser auf 80 °C erwärmt. Unter Rühren wurden dieser Lösung 4,05 g Ethylacrylat und 0.94 g einer 4,3 %igen methanolischen Azobiscyanvaleriansäurelösung gegeben und 1 h bei 80 °C gerührt. Anschließend wurden während 1 h eine erwärmte Lösung aus 22,95 g Ethylacrylat und 18 g Monomer K-2 in 204,3 ml Methanol sowie 9,5 g einer 4,3 %igen methanolischen Azobiscyanvaleriansäurelösung zugetropft. Nach weiteren 2 h Rühren bei 80 °C wurden das Methanol aus dem Reaktionsgemisch abgedampft und der erhaltene Latex filtriert. Es wurde ein feinteiliger Latexkuppler mit 10,6 % Feststoff erhalten.

Polymerkuppler P-2

Copolymer aus Monomer K-7 und Butylacrylat

Unter Stickstoff wurde eine Lösung aus 0,63 g Oleylmethyltaurid in 121 g Wasser auf 80 °C erwärmt. Dazu wurden 1,4 g Butylacrylat und 0,33 g einer 4,3 %igen methanolischen Lösung von Azobiscyanvaleriansäure gegeben und 1 h bei 80 °C gerührt. Anschließend wurde eine Mischung aus 8 g Butylacrylat, 36,3 ml Methanol und 23,2 g einer 17,3 %igen methanolischen Lösung von K-7 sowie 2,8 g einer 4,3 %igen methanolischen Azobiscyanvaleriansäure-Lösung der Reaktionslösung zugesetzt. Es wurde 2 h bei 80 °C nachgerührt und anschließend das Methanol durch Destillation abgetrennt. Es wurde ein feinteiliger Latexkuppler erhalten. Feststoff: 12,0 %.

Polymerkuppler P-3

Copolymer aus Monomer K-2 und Butylacrylat

Die Herstellung erfolgte wie bei Polymerkuppler P-1 beschrieben, aber anstelle von Ethylacrylat wurde Butylacrylat verwendet und anstelle der methanolischen Azobiscyanvaleriansäure wurde eine wäßrige Kaliumperoxidisulfatlösung eingesetzt. Es wurde ein feinteiliger Latexkuppler mit einem Feststoffgehalt von 12,6 % erhalten.

Polymerkuppler P-4 bis P-15

Unter Verwendung der vorstehend beschriebenen kupplerhaltigen Monomere K wurden die in der folgenden Tabelle 1 beschriebenen Polymerkuppler auf die gleiche Weise wie für die Copolymere in den vorstehenden Beispielen beschrieben hergestellt.

Für die Comonomere C werden folgende Abkürzungen verwendet:

| | |
|---|---|
| BA | Butylacrylat |
| EA | Ethylacrylat |
| EHA | Ethylhexylacrylat |
| MA | Methylacrylat |
| BM | Butylmethacrylat |
| PPA | i-Propylacrylamid |
| AMPS | 2-Acrylamido-2-methylpropansulfonsäure |

**Tabelle 1**

| Polymerkuppler | Monomer K | Gew.-% | Monomer C | Gew.-% |
|---|---|---|---|---|
| P-4 | K-2 | 60 | BM | 40 |
| P-5 | K-2 | 50 | MA/BA 1:1 | 50 |
| P-6 | K-3 | 40 | BA | 60 |
| P-7 | K-3 | 30 | EHA | 70 |
| P-8 | K-7 | 60 | BA | 40 |
| P-9 | K-7 | 50 | BA/PPA 8:2 | 50 |
| P-10 | K-7 | 55 | EA | 45 |
| P-12 | K-11 | 40 | BA | 60 |
| P-13 | K-11 | 70 | BA | 30 |

Das erfindungsgemäße farbfotografische Aufzeichnungsmaterial enthält mindestens eine lichtempfindliche Silberhalogenidemulsionsschicht und vorzugsweise eine Abfolge mehrerer solcher lichtempfindlichen Silberhalogenidemulsionsschichten und gegebenenfalls dazwischen angeordneten nicht lichtempfindlichen Bindemittelschichten, wobei mindestens einer der vorhandenen lichtempfindlichen Silberhalogenidemulsionsschichten ein polymerer Purpurkuppler der vorliegenden Erfindung zugeordnet ist.

Die in den lichtempfindlichen Schichten verwendeten lichtempfindlichen Silberhalogenidemulsionen können als Halogenid Chlorid, Bromid und Iodid bzw. Mischungen davon enthalten. Beispielsweise kann der Halogenidanteil wenigstens einer Schicht zu 0 bis 12 mol-% aus Iodid, zu 0 bis 50 mol-% aus Chlorid und zu 50 bis 100 mol-% aus Bromid bestehen. In bestimmten Ausführungsformen handelt es sich um überwiegend kompakte Kristalle, die z.B. kubisch oder oktaedrisch sind oder Übergangsformen aufweisen. Sie lassen sich dadurch kennzeichnen, daß sie im wesentlichen eine Dicke von mehr als 0,2 µm aufweisen. Das durchschnittliche Verhältnis von Durchmesser zu Dicke ist bevorzugt kleiner als 8:1, wobei gilt, daß der Durchmesser eines Kornes definiert ist als der Durchmesser eines Kreises mit einem Kreisinhalt entsprechend der projizierten Fläche des Kornes. In anderen Ausführungsformen können alle oder einzelne

Emulsionen aber auch im wesentlichen tafelförmige Silberhalogenidkristalle aufweisen, bei denen das Verhältnis von Durchmesser zu Dicke größer als 8:1 ist. Bei den Emulsionen kann es sich um heterodisperse, oder auch um monodisperse Emulsionen handeln, die bevorzugt eine mittlere Korngröße von 0,3 µm bis 1,2 µm aufweisen. Die Silberhalogenidkörner können auch einen geschichteten Kornaufbau aufweisen.

Die Emulsionen können in der üblichen Weise chemisch und oder spektral sensibilisiert sein; sie können auch durch geeignete Zusätze stabilisiert sein. Geeignete chemische Sensibilisatoren, spektrale Sensibilisierungsfarbstoffe und Stabilisatoren sind beispielsweise in Research Disclosure 17643 beschrieben; verwiesen wird insbesondere auf die Kapitel III, IV und VI.

Das erfindungsgemäße farbfotografische Aufzeichnungsmaterial enthält bevorzugt mindestens je eine Silberhalogenidemulsionsschicht für die Aufzeichnung von Licht jedes der drei Spektralbereiche Rot, Grün und Blau. Zu diesem Zweck sind die lichtempfindlichen Schichten in bekannter Weise durch geeignete Sensibilisierungsfarbstoffe spektral sensibilisiert. Blauempfindliche Silberhalogenidemulsionsschichten müssen nicht notwendigerweise einen Spektralsensibilisator enthalten, da für die Aufzeichnung von blauem Licht in vielen Fällen die Eigenempfindlichkeit des Silberhalogenids ausreicht.

Jede der genannten lichtempfindlichen Schichten kann aus einer einzigen Schicht bestehen oder in bekannter Weise, z.B. bei der sogenannten Doppelschichtanordnung, auch zwei oder auch mehr Silberhalogenidemulsionsteilschichten umfassen (DE-C-1 121 470). Üblicherweise sind rotempfindliche Silberhalogenidemulsionsschichten dem Schichtträger näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtempfindliche gelbe Filterschicht befindet. Es sind aber auch andere Anordnungen denkbar. Zwischen Schichten unterschiedlicher Spektralempfindlichkeit ist in der Regel eine nicht lichtempfindliche Zwischenschicht angeordnet, die Mittel zur Unterbindung der Fehldiffusion von Entwickleroxidationsprodukten enthalten kann. Falls mehrere Silberhalogenidemulsionsschichten gleicher Spektralempfindlichkeit vorhanden sind, können diese einander unmittelbar benachbart sein oder so angeordnet sein, daß sich zwischen ihnen eine lichtempfindliche Schicht mit anderer Spektralempfindlichkeit befindet (DE-A-1 958 709, DE-A-2 530 645, DE-A-2 622 922).

Erfindungsgemäße farbfotografische Aufzeichnungsmaterialien enthalten üblicherweise in räumlicher und spektraler Zuordnung zu den Silberhalogenidemulsionsschichten unterschiedlicher Spektralempfindlichkeit Farbkuppler zur Erzeugung der unterschiedlichen Teilfarbenbilder Cyan, Purpur und Gelb, wobei die polymeren Kuppler der vorliegenden Erfindung im allgemeinen einer grünempfindlichen Silberhalogenidemuslionsschicht zugeordnet sind.

Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit jeder der lichtempfindlichen Silberhalogenidemulsionsschichten und die Farbe des aus dem jeweils räumlich zugeordneten Farbkuppler erzeugten Teilfarbenbildes in einer bestimmten Beziehung zueinander stehen, wobei jeder der Spektralempfindlichkeiten (Rot, Grün, Blau) eine andere Farbe des betreffenden Teilfarbenbildes (im allgemeinen z.B. die Farben Cyan, Purpur bzw. Gelb in dieser Reihenfolge) zugeordnet ist.

Jeder der unterschiedlich spektral sensibilisierten Silberhalogenidemulsionsschichtenkann ein oder können auch mehrere Farbkuppler zugeordnet sein. Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Rotempfindlichen Silberhalogenidemulsionsschichten ist folglich bei bevorzugten Ausführungsformen mindestens ein nichtdifundierender Farbkuppler zur Erzeugung des blaugrünen Teilfarbenbildes zugeordnet, in der Regel ein Kuppler vom Phenol- oder α-Naphtholtyp. Grünempfindlichen Silberhalogenidemulsionsschichten ist mindestens ein nichtdiffundierender Farbkuppler zur Erzeugung des purpurnen Teilfarbenbildes zugeordnet, wobei gegebenenfalls zusätzlich zu den erfindungsgemäßen polymeren Purpurkupplern auch monomere (niedermolekulare) Purpurkuppler vom Typ des 5-Pyrazolons, des Indazolons oder des Pyrazoloazols Verwendung finden. Blauempfindlichen Silberhalogenidemulsionsschichten schließlich ist mindestens ein nichtdiffundierender Farbkuppler zur Erzeugung des gelben Teilfarbenbildes zugeordnet, in der Regel ein Farbkuppler mit einer offenkettigen Ketomethylengruppierung. Farbkuppler dieser Art sind in

großer Zahl bekannt und in einer Vielzahl von Patentschriften beschrieben. Beispielhaft sei hier auf die Veröffentlichungen "Farbkuppler" von W. PELZ in "Mitteilungen aus den Forschungslaboratorien der Agfa, Leverkusen/München", Band III, Seite 111 (1961) und von K. VENKATARAMAN in "The Chemistry of Synthetic Dyes", Vol. 4, 341 bis 387, Academic Press (1971), verwiesen.

Bei den erfindungsgemäßen Farbkupplern wie auch bei den übrigen im farbfotografischen Aufzeichnungsmaterial vorhandenen Farbkupplern kann es sich sowohl um übliche 4-Äquivalentkuppler handeln als auch um 2-Äquivalentkuppler, bei denen zur Farberzeugung eine geringere Menge Silberhalogenid erforderlich ist. 2-Äquivalentkuppler leiten sich bekanntlich von den 4-Äquivalentkupplern dadurch ab, daß sie in der Kupplungsstelle einen Substituenten enthalten, der bei der Kupplung abgespalten wird. Zu den 2-Äquivalentkupplern sind sowohl solche zu rechnen, die praktisch farblos sind, als auch solche, die eine intensive Eigenfarbe aufweisen, die bei der Farbkupplung verschwindet bzw. durch die Farbe des erzeugten Bildfarbstoffes ersetzt wird. Letztere Kuppler können ebenfalls zusätzlich in den lichtempfindlichen Silberhalogenidemulsionsschichten vorhanden sein und dort als Maskenkuppler zur Kompensierung der unerwünschten Nebendichten der Bildfarbstoffe dienen. Zu den 2-Äquivalentkupplern sind aber auch die bekannten Weißkuppler zu rechnen, die jedoch bei Reaktion mit Farbentwickleroxidationsprodukten keinen Farbstoff ergeben. Zu den 2-Äquivalentkupplern sind ferner solche Kuppler zu rechnen, die in der Kupplungsstelle einen abspaltbaren Rest enthalten, der bei Reaktion mit Farbentwickleroxidationsprodukten in Freiheit gesetzt wird und dabei eine bestimmte erwünschte fotografische Wirksamkeit entfaltet, z.B. als Entwicklungsinhibitor oder -accelerator. Beispiele für solche 2-Äquivalentkuppler sind die bekannten DIR-Kuppler wie auch DAR-bzw. FAR-Kuppler. Der abgespaltbare Rest kann auch ein Ballastrest sein, so daß bei der Reaktion mit Farbentwickleroxidationsprodukten Kupplungsprodukte z.B. Farbstoffe erhalten werden können, die diffusionsfähig sind oder zumindest eine schwache bzw. eingeschränkte Beweglichkeit aufweisen.

Unter einer schwachen bzw. eingeschränkten Beweglichkeit ist eine Beweglichkeit zu verstehen, die so bemessen ist, daß die Konturen der bei der chromogenen Entwicklung gebildeten diskreten Farbstoffflecken verlaufen und ineinander verschmiert werden. Dieses Ausmaß der Beweglichkeit ist einerseits zu unterscheiden von dem üblichen Fall der völligen Unbeweglichkeit in fotografischen Schichten, der in der herkömmlichen fotografischen Aufzeichnungsmaterialien für die Farbkuppler bzw. die daraus hergestellten Farbstoffe angestrebt wird um eine möglichst hohe Schärfe zu erzielen, und andererseits von dem Fall der völligen Beweglichkeit der Farbstoffe, der beispielsweise bei Farbdiffusionsverfahren angestrebt wird. Die letztgenannten Farbstoffe verfügen meist über mindestens eine Gruppe, die sie im alkalischen Medium löslich macht. Das Ausmaß der erfindungsgemäß angestrebten schwachen Beweglichkeit kann gesteuert werden durch Variation von Substituenten um beispielsweise die Löslichkeit im organischen Medium des Ölbildners oder die Affinität zur Bindemittelmatrix in gezielter Weise zu beeinflussen.

Für die erfindungsgemäßen Aufzeichnungsmaterialien eignen sich die üblichen Schichtträger, z.B. Träger aus Celluloseestern, z.B. Celluloseacetat und aus Polyestern. Geeignet sind ferner Papierträger, die gegebenenfalls beschichtet sein können z.B. mit Polyolefinen, insbesondere mit Polyethylen oder Polypropylen. Verwiesen wird diesbezüglich auf die oben angegebene Research Disclosure 17643, Kapitel XVII.

Als Schutzkolloid bzw. Bindemittel für die Schichten des Aufzeichnungsmaterials sind die üblichen hydrophilen filmbildenden Mittel geeignet, z.B. Proteine, insbesondere Gelatine. Begußhilfsmittel und Weichmacher können verwendet werden. Verwiesen wird auf die in der oben angegebenen Research Disclosure 17643, Kapitel IX, XI und XII.

Die Schichten des fotografischen Materials können in der üblichen Weise gehärtet sein, beispielsweise mit Härtern, die mindestens zwei reaktive Oxiran-, Aziridin-oder Acryloylgruppen enthalten. Weiterhin ist es auch möglich, die Schichten gemäß dem in DE-A-22 18 009 beschriebenen Verfahren zu härten. Es ist ferner möglich, die fotografischen Schichten bzw. die farbfotografischen Mehrschichtenmaterialien mit Härtern der Diazin-Triazin-oder 1,2-Dihydrochinolin-Reihe zu härten oder mit Härtern vom Vinylsulfon-Typ. Weitere geeignete Härtungsmittel sind aus DE-A-24 39 551, DE-A-22 25 230, DE-A-22 17 672 wie auch aus Research Disclosure 17 643, Kapitel X bekannt.

Weitere geeignete Zusätze werden in der Research Disclosure 17 643 und in "Product Licensing Index" von Dezember 1971, Seiten 107-110, angegeben.

Geeignete Farbentwicklersubstanzen für das erfindungsgemäße Material sind insbesondere solche vom p-Phenylendiamintyp, z.B. 4-Amino-N,N-diethyl-anilinhydrochlorid, 4-Amino-3-methyl-N-ethyl-N-$\beta$-(methansulfonamido)-ethyl-anilinsulfathydrat, 4-Amino-3-methyl-N-ethyl-N-$\beta$-hydroxyethylanilinsulfat, 4-Amino-N-ethyl-N-(2-methoxy-ethyl)-m-toluidin-di-p-toluolsulfonsäure und N-Ethyl-N-$\beta$-hydroxyethyl-p-phenylendiamin. Weitere brauchbare Farbentwickler sind beispielsweise beschrieben in J.Amer.Chem.Soc. 73, 3100 (1951) und in G. Haist, Modern Photographic Processing, 1979, John Wiley and Sons, New York, Seiten 545 ff.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren, insbesondere z.B. Ethylendiamintetraessigsäure, Nitrilotriessigsäure, Iminodiessigsäure, N-Hydroxyethylethylendiamintriessigsäure, Alkyliminodicarbonsäuren und von entsprechenden Phosphonsäuren. Geeignet als Bleichmittel sind weiterhin Persulfate.

Beispiel 1

Die erfindungsgemäßen Polymerkuppler P-1, P-2, P-3, P-5, P-9 und P-10 und die polymeren Vergleichskuppler A und B wurden verschiedenen Proben einer Silberhalogenidemulsion zugemischt, die dem eingebrachten Farbkuppler entsprechend für Grün sensibilisiert war. Die verwendete Silberhalogenidgelatine-Emulsion bestand aus 75 g Silberbromidiodid (Iodidgehalt 3 mol-%) und 72 g Gelatine bezogen auf 1 kg Emulsion.

Die so präparierten Emulsionen wurden auf mit einer Haftschicht versehene Cellulosetriacetatschichtträger aufgetragen und getrocknet.

Fotografische Prüfung:

Die einzelnen Proben wurden mittels eines Sensitometers belichtet und danach unter Verwendung des folgenden Farbentwicklers verarbeitet.

Farbentwickler

| | |
|---|---|
| Wasser dest. | 800 g |
| Hydroxyethandisphosphonsäure-di-Na-Salz | 2 g |
| Ethylendiamintetraessigsäure-di-Na-Salz | 2 g |
| Kaliumcarbonat | 34 g |
| Natriumhydrogencarbonat | 1,55 g |
| Natriumdisulfit | 0,28 g |
| Natriumsulfit | 3,46 g |
| Kaliumbromid | 1,34 g |
| Hydroxylaminsulfat | 2,4 g |
| N-Ethyl-N-($\beta$-hydroxy)-ethyl-4-amino-3-ethylanilinsulfat | 4,7 g |
| Wasser dest. bis 1000 ml | |

Verarbeitung

| | Verarbeitung [min] ($25^{o}$ C) |
|---|---|
| Farbentwickler | 10 |
| Stoppbad | 4 |
| Zwischenwässerung | 5 |
| Bleichbad | 5 |
| Zwischenwässerung | 5 |
| Fixierbad | 5 |
| Schlußwässerung | 10 |

Bei den Stopp-, Bleich- und Fixierbädern handelt es sich um übliche Badzusammensetzungen. Es wurde ein formalinfreies Schlußbad verwendet.

Bestimmt wurde das Absorptionsmaximum λmax und die maximale Farbdichte Dmax (Tabelle 2).

Tabelle 2

| Polymerkuppler | Dmax | λmax |
|---|---|---|
| A (Vergleich) | 0,62 | 545 |
| B (Vergleich) | 0,51 | 547 |
| 1 | 2,75 | 548 |
| 2 | 2,88 | 548 |
| 3 | 2,93 | 550 |
| 5 | 3,01 | 548 |
| 9 | 2,67 | 547 |
| 10 | 2,95 | 548 |

Aus den ergebnissen ist ersichtlich, daß nur mit den erfindungsgemäßen Latexkupplern von Pyrazoloazol Typ gute Maximaldichten erreicht werden. Die Vergleichspolymeren A und B sind wegen der geringen Farbdichten für die Praxis unbrauchbar.

Als Vergleichskupplerpolymere wurden die Pyrazoloazolpolymeren A und B verwendet (vgl. US-A-4 576 910 und EP-A-0-133262).

Vergleichspolymer A

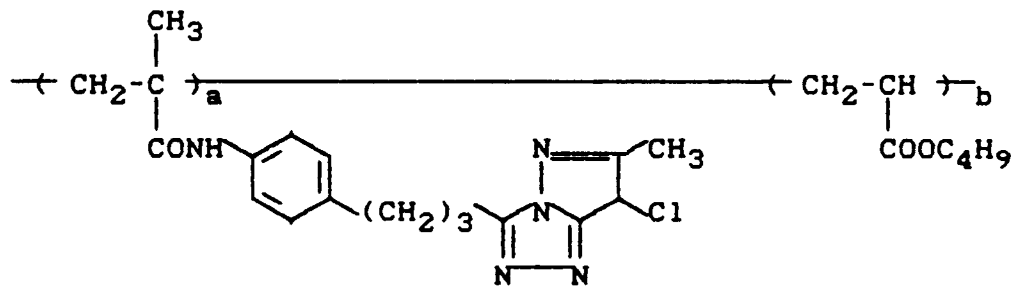

a/b = 30/70 Gew.-%

Vergleichsbeispiel B

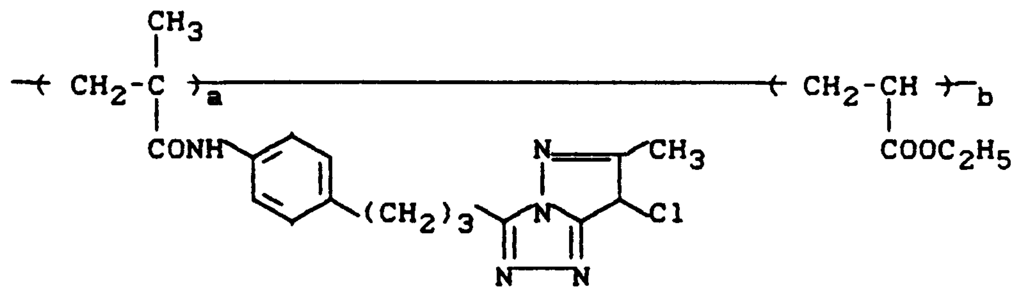

a/b = 40/60 Gew-%.

**Patentansprüche**

**1.** Purpurkuppler mit mindestens einer kupplungsfähigen Struktureinheit der Formel I

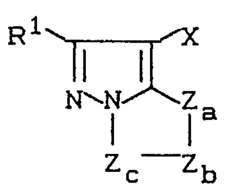

I

worin bedeuten

- $R^1$ H, Alkyl, Aralkyl oder Aryl;
- X H oder eine durch Kupplung freisetzbare Gruppe;
- $Z_a$, $Z_b$, $Z_c$ eine gegebenenfalls substituierte Methingruppe, =N- oder -NH-, wobei entweder die Bindung $Z_a$ - $Z_b$ oder die Bindung $Z_b$ - $Z_c$ eine Doppelbindung und die jeweils andere Bindung eine Einfachbindung ist;

und worin mindestens $R^1$ oder X oder ein Substituent an einer durch $Z_a$, $Z_b$ oder $Z_c$ dargestellten Methingruppe eine ethylenisch ungesättigte polymerisierbare Gruppe in gegebenenfalls polymerisierter Form enthält,
die direkt oder über ein Bindeglied der Formel

$$-(L^0)_k-(L^1)_l-(L^2)_m-(L^3)_n-(L^4)_o-(L^5)_p-(L^6)_q-(L^7)_r-$$

an einen der beiden heterocyclischen Ringe gebunden ist, worin $L^0$ den dem heterocyclischen Ring und $L^7$ den der ethylenisch ungesättigten Gruppe zunächst gelegenen Teil des Bindegliedes bezeichnen und worin bedeuten (gleich oder verschieden):

$L^0$, $L^2$, $L^4$, $L^6$ -O-, -NR-, -NRCO-, -CONR-, -NRSO$_2$-, -SO$_2$NR-, -COO-, -O-CO-, -NR-CO-NR-, -O-CO-NR-, -NH-COO- (mit R = H oder Alkyl, das gegebenenfalls mit Carboxyl substituiert sein kann.);

$L^1$, $L^3$, $L^5$, $L^7$ Alkylen, Aralkylen, Arylen, gegebenenfalls mit Carboxy substituiert;

k,l,m,n,o,p,q,r jeweils 0 oder 1, wobei gilt, daß

$$l - m + n - o + p - q = 0,$$

dadurch gekennzeichnet, daß mindestens $R^1$ oder X oder ein Substituent an einer durch $Z_a$, $Z_b$ oder $Z_c$ dargestellten Methingruppe eine Carboxylgruppe enthält, mit der Maßgabe, daß wenn die ethylenisch ungesättigte polymerisierbare Gruppe in $R^1$ oder in einem Substituenten an einer durch $Z_a$, $Z_b$ oder $Z_c$ dargestellten Methingruppe enthalten ist, mindestens eins von $L^0$, $L^2$, $L^4$ und $L^6$ ein N-Atom mit einer daran gebundenen Carboxyalkylgruppe enthält oder ein C-Atom der ethylenisch ungesättigten polymerisierbaren Gruppe mit einer Carboxygruppe oder einer Carboxyalkylgruppe substituiert ist.

2. Purpurkuppler nach Anspruch 1, dadurch gekennzeichnet, daß er durch Polymerisation erhalten wurde und mindestens 20 Gew.-% an polymerisierten Struktureinheiten der Formel I enthält.

3. Purpurkuppler nach Anspruch 2, gekennzeichnet durch einen Gehalt von 30-70 Gew.-% an polymerisierten Struktureinheiten der Formel I und 70-30 Gew.-% an polymerisierten Struktureinheiten eines oder mehrerer damit copolymerisierbarer Monomeren.

4. Farbfotografisches Aufzeichnungsmaterial mit mindenstens einer lichtempfindlichen Silberhalogenidemulsionsschicht und mindestens einem Purpurkuppler nach einem der Ansprüche 1-3.

## Claims

1. Magenta couplers containing at least one couplable structural unit corresponding to formula I:

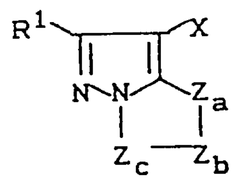

I

in which

$R^1$ is H, alkyl, aralkyl or aryl;

X is H or a group releasable by coupling;

$Z_a$, $Z_b$, $Z_c$ represent an optionally substituted methine group, =N- or -NH-; either the bond $Z_a$ - $Z_b$ or the bond $Z_b$ - $Z_c$ being a double bond and the other bond being a single bond;

and in which at least $R^1$ or X or a substituent at a methine group $Z_a$, $Z_b$ or $Z_c$ contains an ethylenically unsaturated polymerizable group in optionally polymerized form which is attached to one of the two heterocyclic rings either directly or by a binding link corresponding to the formula:

$$-(L^0)_k-(L^1)_l-(L^2)_m-(L^3)_n-(L^4)_o-(L^5)_p-(L^6)_q-(L^7)_r$$

in which $L^0$ is that part of the binding link which is situated nearest the heterocyclic ring and $L^7$ is that part of the binding link which is situated nearest the ethylenically unsaturated group and in which the symbols $L^0$ to $L^7$, which may be the same or different, have the following meanings:

$L^0$, $L^2$, $L^4$, $L^6$ = -O-, -NR-, -NRCO-, -CONR-, -NRSO$_2$-, -SO$_2$NR-, -COO-, -O-CO-, -NR-CO-NR-, -O-CO-NR-, -NH-COO- (where R = H or alkyl, optionally substituted by carboxyl);

$L^1$, $L^3$, $L^5$, $L^7$ represent alkyl, aralkylene, arylene optionally substituted by carboxy;
k,l,m,n,o,p,q,r are each 0 or 1, with the proviso that

$$l - m + n - o + p - q = 0,$$

characterized in that at least $R^1$ or X or a substituent at a methine group $Z_a$, $Z_b$ or $Z_c$ contains a carboxyl group with the proviso that, when the ethylenically unsaturated polymerizable group is present in $R^1$ or in a substituent at a methine group $Z_a$, $Z_b$ or $Z_c$, at least one of $L^0$, $L^2$, $L^4$ and $L^6$ contains a nitrogen atom with a carboxyalkyl group attached thereto or a carbon atom of the ethylenically unsaturated polymerizable group is substituted by a carboxy group or by a carboxyalkyl group.

**2.** A magenta coupler as claimed in claim 1, characterized in that it is obtained by polymerization and contains at least 20% by weight polymerized structural units corresponding to formula I.

**3.** A magenta coupler as claimed in claim 2, characterized by a content of 30 to 70% by weight polymerized structural units corresponding to formula I and 70 to 30% by weight polymerized structural units of one or more monomers copolymerizable therewith.

**4.** A colour photographic recording material containing at least one photosensitive silver halide emulsion layer and at least one magenta coupler according to any of claims 1 to 3.

**Revendications**

**1.** Coupleur magenta présentant au moins un motif structural apte au couplage, de formule I

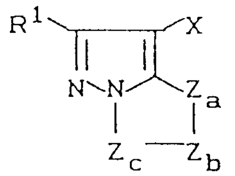

I

dans laquelle

$R^1$ représente H, un groupe alkyle, aralkyle ou aryle ;
X représente H ou un groupe libérable par couplage
$Z_a$, $Z_b$, $Z_c$ représentent un groupe méthine éventuellement substitué =N-, ou -NH-, l'une des liaisons $Z_a$ -$Z_b$ et $Z_b$ - $Z_c$ étant une liaison double et l'autre de ces deux liaisons étant une liaison simple ;

et dans laquelle l'un au moins de $R^1$ et X ou un substituant d'un groupe méthine représente par $Z_a$, $Z_b$ ou $Z_c$ contenant un groupe polymérisable à non-saturation éthylénique sous la forme éventuellement polymérisée,
qui est lié directement ou par l'intermédiaire d'un membre de liaison de formule :

$$-(L^0)_k-(L^1)_l-(L^2)_m-(L^3)_n-(L^4)_o-(L^5)_p-(L^6)_q-(L^7)_r-$$

à l'un des deux noyaux hétérocycliques, $L^0$ désignant la partie du membre de liaison la plus proche du noyau hétérocyclique et $L^7$ désignant la partie du membre de liaison la plus proche du groupe à non-saturation éthylénique et dans laquelle (avec des valeurs identiques ou différentes) :

$L^0$, $L^2$, $L^4$, $L^6$ représentent -O-, -NR-, -NRCO-, -CONR-, $-NRSO_2-$, $-SO_2NR-$, -COO-, -O-CO-, -NR-CO-NR-, -O-CO-NR-, -NH-COO- (avec R = H ou groupe alkyle qui peut être substitué le cas échéant par un radical carboxyle) ;
$L^1$, $L^3$, $L^5$, $L^7$ représentent un groupe alkylène, aralkylène, arylène, le cas échéant substitué avec un radical alkoxy ;
k,l,m,n,o,p,q,r ont chacun la valeur 0 ou 1, de telle sorte que l - m + n - o + p - q ait la valeur 0,

caractérisé en ce que l'un au moins de $R^1$ ou X ou un substituant d'un groupe méthine représenté par $Z_a$, $Z_b$ ou $Z_c$ contient un groupe carboxyle, sous réserve que lorsque le groupe polymérisable à non-saturation éthylénique est contenu dans $R^1$ ou dans un substituant d'un groupe méthine représenté par $Z_a$, $Z_b$ ou $Z_c$, l'un au moins de $L^0$, $L^2$, $L^4$ et $L^6$ contienne un atome d'azote avec un groupe

carboxyalkyle lié à cet atome, ou un atome de carbone du groupe polymérisable à non-saturation éthylénique soit substitué avec un groupe carboxy ou avec un groupe carboxyalkyle.

**2.** Coupleur magenta suivant la revendication 1, caractérisé en ce qu'il a été obtenu par polymérisation et en ce qu'il contient au moins 20 % en poids de motifs structuraux polymérisés de formule I.

**3.** Coupleur magenta suivant la revendication 2, caractérisé par une teneur de 30 à 70 % en poids en motifs structuraux polymérisés de formule I et en une teneur de 70 à 30 % en poids de motifs structuraux polymérisés d'un ou plusieurs monomères copolymérisables avec eux.

**4.** Support d'enregistrement pour la photographie en couleur comprenant au moins une couche d'émulsion photosensible d'halogénure d'argent et au moins un coupleur magenta suivant l'une des revendications 1 à 3.